# FASCICULE DE BREVET EUROPEEN

(11) **EP 3 084 505 B1**
(45) Date de publication et mention de la délivrance du brevet: **19.06.2019**
(21) Numéro de dépôt: 14824808.1
(22) Date de dépôt: 17.12.2014
(51) Int. Cl.: G02B 23/24, G02B 23/26, A61B 1/00

(54) **DISPOSITIF D'IMAGERIE STRUCTURÉE TRIDIMENSIONNELLE À GUIDES D'IMAGES RÉVERSIBLES**
STRUKTURIERTE DREIDIMENSIONALE BILDGEBUNGSVORRICHTUNG MIT UMKEHRBAREN BILDFÜHRUNGEN
STRUCTURED THREE-DIMENSIONAL IMAGING DEVICE WITH REVERSIBLE IMAGE GUIDES

(30) Priorité: 17.12.2013 FR 1362746
(43) Date de publication de la demande: 26.10.2016
(73) Titulaire: Université Technologie de Compiègne-UTC, 60200 Compiègne (FR); CNRS Centre National de la Recherche Scientifique, 75016 Paris (FR); Institut National des Sciences Appliquées de Lyon, 69100 Villeurbanne (FR); Ecole Centrale de Lyon, 69130 Ecully (FR); Université Claude Bernard Lyon 1, 69100 Villeurbanne (FR)
(72) Inventeur: LAMARQUE, Frédéric, F-60200 Compiegne (FR); DUPONT, Erwan, F-60170 Tracy Le Val (FR); PRELLE, Christine, F-60200 Compiegne (FR); PETIT, Laurent, 60880 Le Meux (FR); REDARCE, Tanneguy, F-69500 Bron (FR)
(74) Mandataire: Regimbeau
(86) Numéro de dépôt international: PCT/EP2014/078197
(87) Numéro de publication internationale: WO 2015/091631

(56) Documents cités:
- WO-A1-94/13189
- WO-A1-2012/076128
- WO-A1-2013/033811
- WO-A2-00/03272
- WO-A2-2011/014687
- DE-A1- 3 818 104

## Description

### DOMAINE GENERAL

L'invention concerne un dispositif d'imagerie à vision tridimensionnelle.

### ETAT DE L'ART

Des dispositifs d'image à vision tridimensionnelle sont utilisés dans de multiples domaines. Un de ces domaines est celui de l'endoscopie.

L'endoscopie est une méthode d'exploration et d'imagerie médicale ou industrielle qui permet de visualiser l'intérieur de conduits ou de cavités inaccessibles à l'oeil.

L'endoscopie peut notamment être utilisée, soit pour un diagnostic, soit pour traiter une maladie dans le domaine médical, par coloscopie par exemple.

En général, l'endoscopie est réalisée de manière bidimensionnelle; il existe cependant des endoscopes à vision tridimensionnelle. Différentes méthodes d'acquisition d'images existent afin d'obtenir une mesure tridimensionnelle d'un objet au moyen d'un endoscope.

A titre d'exemple, on peut citer les publications DE3818104 A1 et WO2013/033811 A1.

Parmi les procédés connus, certains fonctionnent par vision dite « passive ». Selon ces procédés, pour reproduire une perception du relief, on acquiert simultanément par deux voies distinctes deux images décalées de l'objet.

Un autre type de procédé connu consiste à mettre en oeuvre une vision dite « active », par l'intermédiaire de projection d'une lumière structurée. Une lumière est dite structurée lorsqu'elle est spatialement modulée en intensité. Elle constitue ainsi dans l'espace un motif lumineux tel que des franges, une grille ou d'autres formes plus complexes.

Une projection de lumière structurée est particulièrement utile pour acquérir une information dimensionnelle concernant un objet à observer. En balayant cet objet avec une lumière structurée et en acquérant les déformations de la lumière réfléchie sous un angle déterminé, il est possible de déduire la géométrie tridimensionnelle de l'objet.

Le balayage de l'objet s'effectue selon diverses méthodes, notamment par modification temporelle du contenu de la lumière structurée projetée. La modification temporelle de la lumière structurée consiste à éclairer spatialement la surface de l'objet à observer avec un motif déterminé, puis à modifier le motif au cours du temps. Par exemple, au cours de l'acquisition, un premier motif à franges horizontales et un deuxième motif de franges verticales orthogonales aux franges du premier motif sont successivement générés et projetés.

Simultanément, un système d'acquisition reçoit les images constituées de la lumière réfléchie par l'objet à observer et peuvent ensuite reconstituer la surface en trois dimensions dudit objet, grâce à une analyse de ces images, et un algorithme de reconstruction (par exemple un algorithme de type à décalage de phase).

Certains endoscopes à vision active connus comprennent à leur extrémité distale des composants optoélectroniques tels qu'une caméra ou un générateur de lumière structurée.

Or, un endoscope doit avoir un diamètre suffisamment petit pour pouvoir être inséré dans un conduit ou une cavité étroite. La conception de tels endoscopes à vision active implique donc en général une recherche de miniaturisation des ces composants optoélectroniques afin de minimiser le diamètre de l'endoscope.

Cependant, cette recherche de miniaturisation implique elle-même une limitation des performances optiques de l'endoscope (par exemple, en termes de résolution) et/ou une augmentation du coût de sa fabrication.

Par ailleurs, la plupart des endoscopes de type connu ne permettent pas de reconstituer certaines portions d'objets tridimensionnels de relief complexe lorsque l'endoscope est placé à une position déterminée, notamment les nombreux endoscopes projetant de la lumière structurée selon un unique angle d'incidence. D'une part des zones telles que des anfractuosités peuvent rester non-illuminées par l'endoscope. D'autre part, des reflets spéculaires non désirés peuvent parasiter l'image structurée réfléchie vers l'endoscope.

### PRESENTATION DE L'INVENTION

L'invention vise à permettre d'imager des surfaces à observer de relief complexe tout en réduisant les contraintes de miniaturisation imposées au dispositif utilisé.

L'invention vise également à augmenter la quantité d'information représentative d'un objet observé par un dispositif d'imagerie.

Il est à cet égard proposé, selon un premier aspect, un dispositif d'imagerie comprenant :
- au moins un générateur de lumière structurée,
- un premier guide d'images d'émission pour acheminer par voie montante la lumière structurée depuis le générateur vers un objet à observer,
- un deuxième guide d'images de retour pour acheminer par voie descendante la lumière réfléchie par ledit objet à observer, jusqu'à un système d'acquisition de ladite lumière réfléchie,
dans lequel chacun des deux guides d'images est apte à acheminer la lumière structurée par voie montante et la lumière réfléchie par voie descendante. Le dispositif comprend un aiguilleur optique configurable:
- dans un premier mode de fonctionnement dans lequel il dirige sélectivement la lumière structurée issue du générateur vers le premier guide d'images, par voie montante, et dirige dans le même temps sélectivement la lumière réfléchie par l'objet depuis le deuxième guide d'images, par voie descendante, jusqu'au système d'acquisition,
- dans un deuxième mode de fonctionnement dans lequel il dirige sélectivement la lumière structurée issue du générateur vers le deuxième guide d'images, par voie montante, et diriger dans le même temps sélectivement la lumière réfléchie par l'objet depuis le premier guide d'images, par voie descendante, jusqu'au système d'acquisition, et
- dans un troisième mode de fonctionnement dans lequel il dirige simultanément de la lumière en provenance des deux guides d'images par voie descendante jusqu'au système d'acquisition.

Les premier et deuxième mode de fonctionnement d'un tel dispositif constituent deux modes de vision active alternatifs, et permettent d'obtenir des informations visuelles différentes sur un objet observé d'enveloppe tridimensionnelle complexe. En effet, les zones de l'objet observé sur lesquelles la lumière structurée est réfléchie lorsque le premier guide d'images est utilisé en voie montante (premier mode de vision active), et lorsque le deuxième guide d'images est utilisé en voie montante (deuxième mode de vision active) ne sont pas forcément identiques.

Le troisième mode de fonctionnement du dispositif constitue un mode de stéréovision passive permettant l'acquisition d'informations tridimensionnelles complémentaires à celles acquises au moyen des deux modes de fonctionnement actifs.

Ainsi, grâce aux trois modes de fonctionnements dans lesquels l'aiguilleur optique peut être configuré, un plus grand nombre d'informations visuelles peut ainsi être collecté avec seulement deux guides d'images, permettant de reconstruire l'enveloppe de l'objet en trois dimensions.

Le dispositif selon ce premier aspect de l'invention peut également comprendre les caractéristiques suivantes, prises seules ou bien en combinaison lorsque cela est techniquement possible.

Le dispositif comprend une source lumineuse secondaire adaptée pour éclairer l'objet.

Le dispositif comprend une fibre optique pour acheminer de la lumière émise par la source secondaire vers l'objet.

Le dispositif comprend un embout distal configuré pour diffuser de la lumière acheminée par chacun des deux guides d'images sur l'objet à observer et rediriger vers chacun des deux guides d'images de la lumière réfléchie par ledit objet.

Le dispositif comprend deux bras distaux, chaque bras distal étant configuré pour diffuser la lumière acheminée par l'un des guides d'images et rediriger vers ce guide d'image la lumière réfléchie par ledit objet, les deux bras étant mobiles l'un par rapport à l'autre de sorte à former un angle de réflexion variable avec l'objet à observer en fonction de la distance de l'objet par rapport aux deux bras.

Chaque guide d'images comprend un faisceau de fibres optiques.

Chaque guide d'images comprend un agencement de miroirs et/ou de lentilles.

L'aiguilleur optique comprend un miroir primaire disposé entre le générateur et les deux guides d'images, le miroir primaire étant adapté pour permettre une redirection sélective de lumière structurée en provenance du générateur vers un premier axe optique menant au premier guide d'images ou vers un deuxième axe optique menant au deuxième guide d'images.

Le générateur est configuré pour émettre de la lumière structurée selon le premier axe menant au premier guide d'images, et le miroir primaire est mobile entre :
- une position active dans laquelle le miroir primaire coupe le premier axe entre les générateur et le premier guide d'images et réoriente vers le deuxième guide d'images la lumière structurée en provenance des générateur selon le deuxième axe, et
- une position passive dans laquelle le miroir primaire ne coupe pas le premier axe.

L'aiguilleur optique comprend un autre miroir agencé pour rediriger la lumière réfléchie par le miroir primaire, lorsqu'il est positionné dans sa position active, sur un autre axe parallèle au premier axe, le premier guide présentant une ouverture proximale alignée sur le premier axe, et le deuxième guide présentant une ouverture proximale alignée sur l'autre axe.

L'aiguilleur optique comprend un premier miroir secondaire un deuxième miroir secondaire, et le premier miroir secondaire est mobile entre :
- une position active dans laquelle le premier miroir secondaire coupe le premier axe entre le générateur et le premier guide d'images, et réoriente vers une caméra de la lumière émanant du premier guide d'images, et
- une position passive dans laquelle le premier miroir secondaire ne coupe pas le premier axe.
De plus, le deuxième miroir secondaire est mobile entre :
- une position active dans laquelle le deuxième miroir secondaire coupe le deuxième axe entre le générateur et le miroir primaire, et réoriente vers le système d'acquisition de la lumière émanant du deuxième guide d'images, et
- une position passive dans laquelle le premier miroir secondaire ne coupe pas le premier axe.

L'aiguilleur optique comprend en outre une unité de commande adaptée pour :
- dans le premier mode de fonctionnement, positionner le miroir primaire et le premier miroir secondaire dans une position passive et le deuxième miroir secondaire dans une position active, et
- dans le deuxième mode de fonctionnement, positionner le miroir primaire et le premier miroir secondaire dans une position active et le deuxième miroir secondaire dans une position passive,
- dans le troisième mode de fonctionnement, positionner les miroirs secondaires dans une position active.

Le miroir primaire et les miroirs secondaires sont chacun mobiles en translation au moyen d'au moins un actionneur électromagnétique bistable.

Le système d'acquisition comprend deux caméras, chaque caméra étant agencée pour recevoir de la lumière en provenance d'un des deux guides d'images.

Le dispositif peut être un endoscope.

L'invention propose selon un deuxième aspect un procédé d'imagerie comprenant les étapes suivantes :
- générer de la lumière structurée,
- acheminer dans un premier guide d'images par voie montante la lumière structurée générée vers un objet à observer,
- acheminer dans un deuxième guide d'images par voie descendante la lumière réfléchie par ledit objet à observer jusqu'à un système d'acquisition de ladite lumière réfléchie,
- acquérir la lumière réfléchie au moyen du système d'acquisition,
le procédé étant caractérisé par les étapes suivantes, chacun des deux guides d'images étant apte à acheminer la lumière structurée par voie montante et la lumière réfléchie par voie descendante:
- diriger sélectivement la lumière structurée générée vers le premier guide d'images, par voie montante, et diriger dans le même temps sélectivement la lumière réfléchie par l'objet depuis le deuxième guide d'images, par voie descendante, jusqu'au système d'acquisition,
- diriger sélectivement la lumière structurée générée vers le deuxième guide d'images, par voie montante, et diriger dans le même temps sélectivement la lumière réfléchie par l'objet depuis le premier guide d'images, par voie descendante, jusqu'au système d'acquisition, et
- diriger simultanément de la lumière en provenance des deux guides d'images par voie descendante jusqu'au système d'acquisition.

L'invention propose selon un troisième aspect un dispositif d'imagerie, tel qu'un endoscope, comprenant :
- au moins un générateur de lumière structurée,
- un premier guide d'images d'émission pour acheminer par voie montante la lumière structurée depuis le générateur jusqu'à un embout distal permettant d'émettre la lumière structurée acheminée sur un objet à observer,
- un deuxième guide d'images de retour pour acheminer par voie descendante la lumière réfléchie par ledit objet à observer, jusqu'à un système d'acquisition de ladite lumière réfléchie,
dans lequel chacun des deux guides d'images est apte à acheminer la lumière structurée par voie montante et la lumière réfléchie par voie descendante, le dispositif comprenant en outre un aiguilleur optique permettant de :
- diriger sélectivement la lumière structurée issue des moyens de génération vers l'un des deux guides d'images, par voie montante et
- diriger dans le même temps sélectivement la lumière réfléchie par l'objet à observer depuis l'autre des deux guides d'images, par voie descendante, jusqu'aux moyens d'acquisition.
Le dispositif selon ce troisième aspect peut également comprendre les caractéristiques optionnelles du dispositif selon le premier aspect de l'invention.

### DESCRIPTION DES FIGURES

D'autres caractéristiques, buts et avantages de l'invention ressortiront de la description qui suit, qui est purement illustrative et non limitative, et qui doit être lue en regard des dessins annexés sur lesquels :
- La figure 1 représente schématiquement un dispositif d'imagerie selon un premier mode de réalisation, configuré dans un premier mode de fonctionnement.
- La figure 2 représente schématiquement le dispositif de la figure 1, configuré dans un deuxième mode de fonctionnement.
- La figure 3 représente schématiquement le dispositif d'imagerie de la figure 1, configuré dans un troisième mode de fonctionnement.
- La figure 4 est une première vue en coupe partielle d'une partie distale d'un dispositif d'imagerie.
- La figure 5 est une deuxième vue en coupe partielle de la partie distale selon un second mode de réalisation.
- La figure 6 représente de façon partielle la partie proximale d'un dispositif d'imagerie, selon un mode de réalisation.
- Les figures 7 à 9 représentent la partie proximale de la figure 6 configurée respectivement dans des premier, deuxième et troisième modes de fonctionnement du dispositif d'imagerie correspondant.
- La figure 10 représente la partie distale d'un dispositif d'imagerie selon un deuxième mode de réalisation de l'invention.
- La figure 11 représente de façon partielle la partie proximale d'un dispositif d'imagerie, selon un mode de réalisation différent de celui représenté en figure 6.
- Les figures 12 à 14 représentent la partie proximale de la figure 11 configurée respectivement dans des premier, deuxième et troisième modes de fonctionnement du dispositif d'imagerie correspondant.

Sur l'ensemble des figures, les éléments similaires portent des références identiques.

### DESCRIPTION DETAILLEE DE L'INVENTION

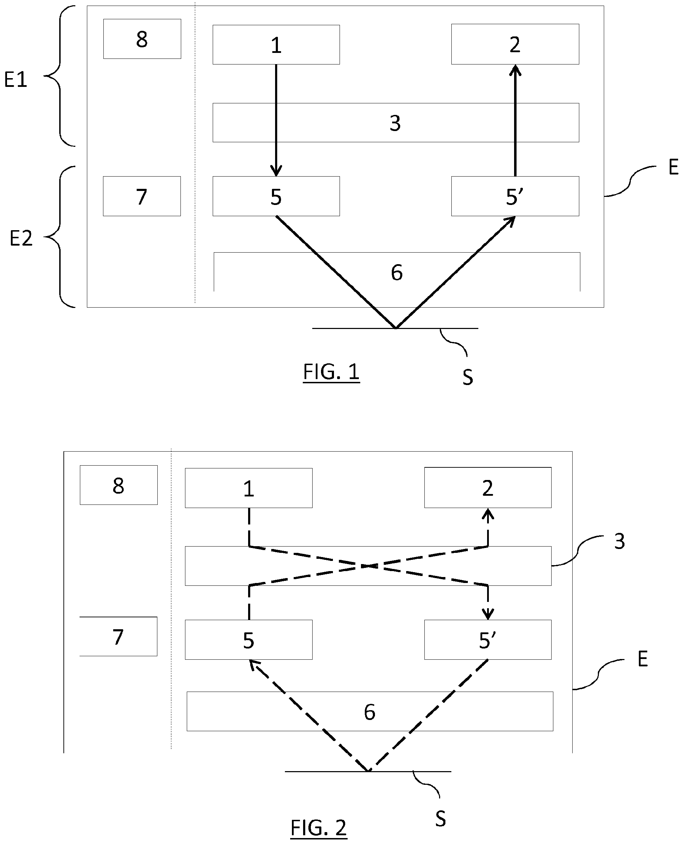

En référence à la figure 1, un dispositif d'imagerie E comprend au moins un générateur 1 de lumière structurée, un système d'acquisition optique 2, un aiguilleur optique 3, et deux guides d'images 5 et 5'.

Le dispositif d'imagerie E comprend une partie proximale E1 comprenant divers dispositifs optoélectroniques, et une partie distale E2.

La partie proximale E1 comprend le générateur 1 de lumière structurée, le système d'acquisition optique 2 et l'aiguilleur optique 3.

La partie distale E2 comprend les deux guides d'images 5 et 5', chaque guide d'images étant adapté pour véhiculer de la lumière structurée.

La partie distale E2 se termine par au moins un embout distal 6 destiné à être placé en regard la surface S d'un objet tridimensionnel à analyser.

On distingue plusieurs types de chemin optiques dans l'endoscope. Par convention, on appelle « voie montante » tout chemin optique véhiculant de la lumière depuis la partie proximale E1 vers la partie distale E2, et par opposition « voie descendante » tout chemin optique véhiculant de la lumière depuis la partie distale E2 vers la partie proximale E1.

Chaque guide d'images 5, 5' est adapté pour acheminer de la lumière structurée émise par le générateur 1, par voie montante, jusqu'à l'embout distal 6 et également pour véhiculer de la lumière réfléchie par un objet observé, par voie descendante, jusqu'au système d'acquisition.

En d'autres termes, chaque guide d'image constitue une interface optique souple ou rigide permettant de transmettre une image d'une de ses extrémités à son autre extrémité.

L'aiguilleur optique 3 assure une fonction d'interface optique entre d'une part le générateur et le système d'acquisition, et d'autre part les guides d'images 5 et 5'. L'aiguilleur optique 3 est adapté pour:
- diriger sélectivement de la lumière structurée générée par le générateur vers l'un des deux guides d'images par voie montante, et
- diriger dans le même temps sélectivement de la lumière réfléchie par l'objet à observer depuis l'autre des deux guides d'images, par voie descendante, vers le système d'acquisition 2.

Le dispositif d'imagerie E comprend en outre une source lumineuse secondaire 8 et une fibre optique 7 raccordée à la source lumineuse secondaire 8 et à l'embout distal 6. La fibre optique 7 est distincte des guides d'images 5 et 5'.

La source lumineuse 8 est adaptée pour émettre de la lumière structurée ou non-structurée. On prendra dans la suite le cas où la source lumineuse 8 émet de la lumière non-structurée.

Trois modes de fonctionnement de du dispositif d'imagerie E sont illustrés en **figures 1 à 3****.**

Dans un premier mode de fonctionnement représenté en **figure 1****,** dit mode de vision active voie gauche, l'aiguilleur optique 3 est configuré pour diriger de la lumière structurée générée par le générateur 1 sélectivement vers le premier guide d'images 5, et est configuré pour rediriger dans le même temps de la lumière en provenance du deuxième guide d'images 5' vers le système d'acquisition 2.

Ainsi, dans le mode de vision active voie gauche, de la lumière structurée (représentée par des flèches pleines) générée par le générateur 1 est dirigée par l'aiguilleur optique 3 sélectivement vers le premier guide d'images 5. La lumière structurée se propage dans le guide 5 jusqu'à l'embout distal 6, sort du dispositif d'imagerie E par l'embout distal 6 et se réfléchit en une zone de la surface S d'un objet tridimensionnel à observer. La lumière structurée ainsi réfléchie pénètre dans le dispositif E par l'embout distal, se propage par voie descendante dans le deuxième guide d'images 5' non utilisé en voie montante, puis est aiguillée par l'aiguilleur optique 3 vers le système d'acquisition 2.

Dans un deuxième mode de fonctionnement illustré en **figure 2****,** dit mode de vision active voie droite, l'aiguilleur optique 3 est configuré pour diriger de la lumière structurée générée par le générateur 1 sélectivement vers le premier guide d'images 5', et est configuré pour rediriger dans le même temps de la lumière en provenance du deuxième guide d'images 5 vers le système d'acquisition 2.

Ainsi, dans le mode de vision active voie droite, de la lumière structurée (représentée par des flèches en pointillés) générée par les le générateur 1 est dirigée par l'aiguilleur optique 3 sélectivement vers le deuxième guide d'images 5'. La lumière structurée se propage dans le deuxième guide 5' jusqu'à l'embout distal 6, sort du dispositif d'imagerie E par l'embout distal 6 et se réfléchit en une zone de la surface S de l'objet tridimensionnel à observer. La lumière structurée ainsi réfléchie pénètre dans le dispositif d'imagerie E par l'embout distal, se propage par voie descendante dans le premier guide d'images 5 non utilisé en voie montante, puis est aiguillée par l'aiguilleur optique 3 vers les le système d'acquisition 2.

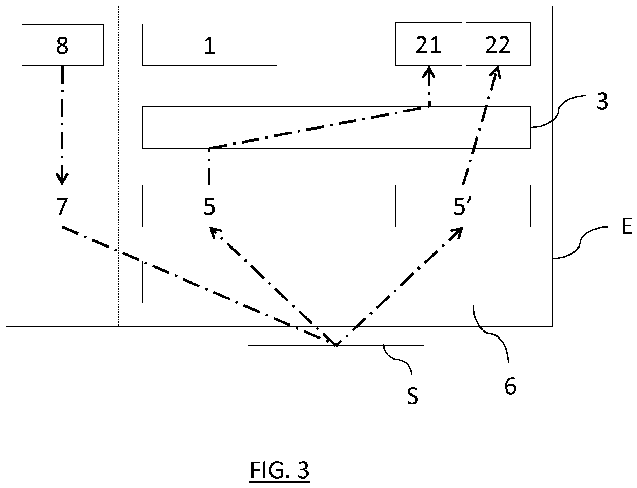

Dans un troisième mode de fonctionnement illustré en **figure 3****,** dit mode de stéréovision passive, l'aiguilleur optique 3 est configuré pour diriger de la lumière structurée ou non structurée en provenance des deux guide d'images 5 et 5' vers le système d'acquisition 2.

Ainsi, dans le mode de stéréovision passive, de la lumière (représentée par des flèches à traits et points alternés) générée par la source lumineuse secondaire 8 se propage dans la fibre optique 7 jusqu'à l'embout distal 6, sort du dispositif d'imagerie E par l'embout distal 6 et se réfléchit en une zone de la surface S d'un objet tridimensionnel à observer. La lumière ainsi réfléchie pénètre à nouveau dans le dispositif d'imagerie par l'embout distal 6, se propage par voie descendante dans les deux guides d'images 5 et 5' non utilisés en voie montante, puis est aiguillée par l'aiguilleur optique 3 vers le système d'acquisition 2.

Dans le mode de stéréovision passive, la fibre optique 7 supplémentaire est utilisée en voie montante tandis que les deux guides d'images 5, 5' sont utilisés en voie descendante.

Le dispositif d'imagerie E peut donc basculer d'un mode de fonctionnement à un autre par simple reconfiguration de l'aiguilleur optique 3. Aucun dispositif optoélectronique n'est utilisé dans l'embout distal 6, l'aiguillage étant compris dans la partie proximale E1 du dispositif d'imagerie E. En particulier, aucun dispositif de balayage de type miroir galvanométrique, ni actionneur piézoélectrique ou électromagnétique n'est requis dans l'embout distal, qui est fixe et non alimenté électriquement.

Le mode de stéréovision passive permet de capturer des nuages de points plus denses sous certaines conditions (si on cherche à mesurer en temps réel, la vision active peut difficilement reconstruire des nuages de points denses, contrairement à la stéréovision passive). La stéréovision passive bénéficie en outre d'un éclairage propre et ne subit donc pas les mêmes effets spéculaires que la vision active (possibilité de reconstruire des zones spéculaires qui posaient problème en vision active). De plus, certaines surfaces absorbant la lumière diffusent mal les motifs en vision active ; la stéréovision passive fournit dans ce cas des images de meilleure qualité.

En revanche, sur des surfaces unies, la vision active fournit de meilleurs résultats que la stéréovision passive.

Le dispositif d'imagerie E proposé permet ainsi d'obtenir des images de bonne qualité pour des surfaces à inspecter de reliefs très différents.

Dans ce qui suit, on considérera un premier mode de réalisation dans lequel le dispositif d'imagerie E est un endoscope, étant entendu que l'invention n'est pas limitée à cette application particulièrement avantageuse.

Dans cette application au domaine de l'endoscopie, la partie distale de l'endoscope E est destinée à être insérée dans le corps d'un patient de façon non-invasive.

### Premier mode de réalisation de partie distale

Dans ce premier mode de réalisation, la partie distale E2 comprend une gaine externe tubulaire définissant un conduit interne. Les guides d'images 5 et 5' sont logés dans le conduit interne et s'étendent entre l'embout distal 6 et l'aiguilleur optique 3 de la partie proximale E1.

Chaque guide d'images 5, 5' comprend par exemple une âme constituée d'un faisceau de fibres optiques parallèles, chaque fibre correspondant à un pixel spécifique d'une image à véhiculer. La pluralité de fibres optiques est entourée d'une gaine de silice et d'un revêtement de protection souple par exemple en plastique.

Le nombre et le diamètre des fibres optiques de chaque guide optique dépendant de la résolution d'image souhaitée. Typiquement, un guide d'images comporte au moins 50000 fibres optiques, par exemple 70000 ; le diamètre de chaque fibre optique est compris entre 1 et 5 micromètres, par exemple 3,5 micromètres ; et le diamètre du guide d'images dans son ensemble est compris entre 1 et 2 millimètres, par exemple 1,3 millimètre.

Bien entendu, les guides d'images à fibres optiques présentés ci-dessus peuvent être remplacés par d'autres types de guides d'images permettant de transmettre une image d'une de leur extrémité à leur autre extrémité.

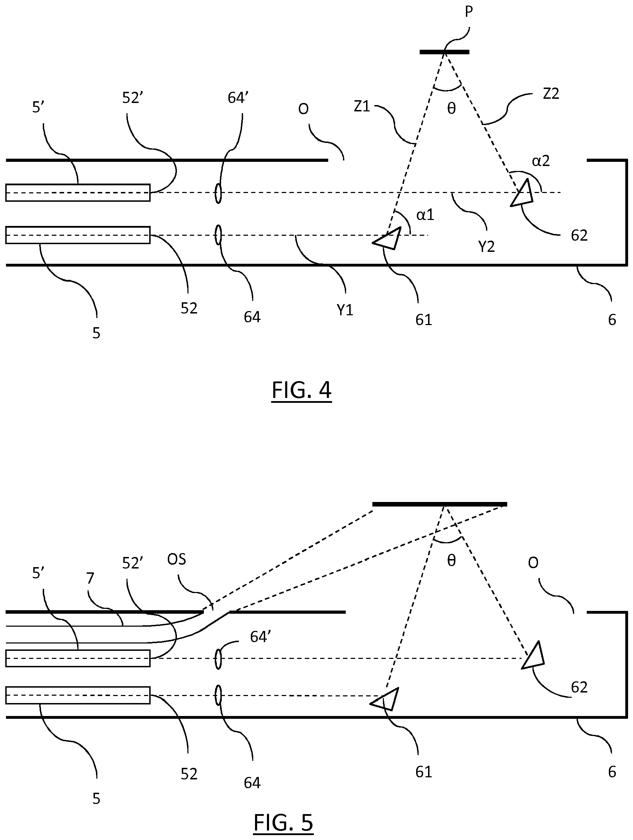

Un mode de réalisation d'embout distal 6 est illustré en figure 4.

L'embout 6 est un corps creux s'étendant selon une direction longitudinale, et comporte une ouverture optique O latérale. Cette ouverture optique O peut par exemple être recouverte d'une surface transparente. Agencer l'ouverture optique O latéralement permet d'augmenter l'angle θ (angle de triangulation) et donc d'améliorer la résolution de mesure axiale.

Les deux guides d'images 5 et 5' comportent chacun une ouverture distale 52, 52' raccordée à l'embout distal 6.

Sont agencés à l'intérieur de l'embout 6 et en regard de l'ouverture latérale O, deux éléments optiques réfléchissants 61 et 62, par exemple des miroirs comportant une surface plane réfléchissante.

Un premier élément optique réfléchissant 61 est placé en regard de l'ouverture distale 52 du premier guide d'images 5, laquelle est centrée sur un premier axe optique Y1 de direction longitudinale.

Le premier élément optique réfléchissant 61 est agencé de façon à réfléchir de la lumière structurée incidente selon l'axe longitudinal Y1 en provenance de l'ouverture distale 52, et la rediriger selon un axe transversal Z1 formant un angle α1 compris entre 45 et 90° par rapport à l'axe longitudinal, par exemple 60°.

Par ailleurs, un deuxième élément optique réfléchissant 62 est placé en regard de l'ouverture distale 52' du deuxième guide d'images 5', laquelle est centrée sur un deuxième axe optique Y2 de direction également longitudinale.

Le deuxième élément optique réfléchissant 62 est agencé de façon à réfléchir de la lumière structurée incidente selon l'axe longitudinal Y2 en provenance de l'ouverture distale 52', et la rediriger selon un axe transversal Z2 formant un angle α2 compris entre 90° et 135° par rapport à l'axe longitudinal, par exemple 110°.

Les positions respectives des éléments optiques réfléchissant 61 et 62 le long de leurs axes longitudinaux et leurs angles de redirections respectifs α1, α2 sont choisies de façon à ce que les axes transversaux Z1, Z2 de ces éléments optiques réfléchissants 61, 62 traversent l'ouverture transversale O et se croisent en au moins un point d'observation P situé à l'extérieur de l'embout 6 au-delà de l'ouverture transversale.

Ainsi, lorsque le point d'observation P rencontre la surface S d'un objet tridimensionnel, de la lumière redirigée par l'un des éléments optique (par exemple 61) est réfléchie au moins partiellement en ce point d'observation P, et est retournée à l'autre des éléments optiques (par exemple 62).

Avantageusement, l'angle entre les axes transversaux Z1 et Z2 est compris entre 50° et 70°, par exemple 60°, plage d'angles particulièrement adaptée pour réaliser une stéréoscopie selon une configuration particulière de l'endoscope qui sera détaillée plus loin dans cette description. Cette plage d'angle offre un bon compromis entre résolution axiale (qui augmente la valeur de cet angle) et la dimension de la zone mesurable (surtout si le point d'observation P est localisé sur une surface non parallèle à l'ouverture O).

Entre l'ouverture distale 52 du premier guide d'images 5 et l'élément optique réfléchissant 61 peut être placé au moins une lentille 64. Similairement, entre l'ouverture distale 52' du deuxième guide d'images 5' et l'élément optique 62 peut être placé au moins une lentille 64'. Chaque lentille 64, 64' sert à focaliser un rayon lumineux en provenance de l'ouverture distale 52, 52' en regard vers le point d'observation P, et à focaliser un rayon réfléchi sur le point P vers l'ouverture distal en regard 52, 52'. Chaque lentille peut être combinée à un diaphragme, qui règle la profondeur de champ (c'est-à-dire la zone de netteté en avant et en arrière du point P).

La distance entre la lentille 64 (respectivement 64') et l'ouverture 52 (respectivement 52') dépend de la focale de la lentille et de la distance entre l'interface 52 (respectivement 52') et le point P.

L'embout 6 ainsi constitué est inerte électriquement et thermiquement. Son rayon moyen par rapport à l'axe longitudinal peut donc être réduit à faible coût, car aucun dispositif électronique n'est requis et n'a alors besoin d'être miniaturisé.

En référence à la figure 5, l'embout 6 comporte par ailleurs une ouverture optique latérale secondaire OS distincte de l'ouverture O. L'ouverture latérale O et l'ouverture latérale secondaire OS peuvent par exemple être centrées sur une même génératrice de la surface externe d'un embout 6 de forme cylindrique, l'ouverture secondaire OS étant ménagée à une position proximale par rapport à l'ouverture O. La fibre optique 7 est raccordée à l'ouverture secondaire OS.

L'ouverture secondaire OS est configurée pour émettre de la lumière non focalisée sur une zone d'un objet en regard de l'ouverture O, la zone éclairée englobant la zone sur laquelle de la lumière structurée émanant de l'un ou l'autre des éléments optiques 61 ou 62 est réfléchie.

### Premier mode de réalisation de partie proximale

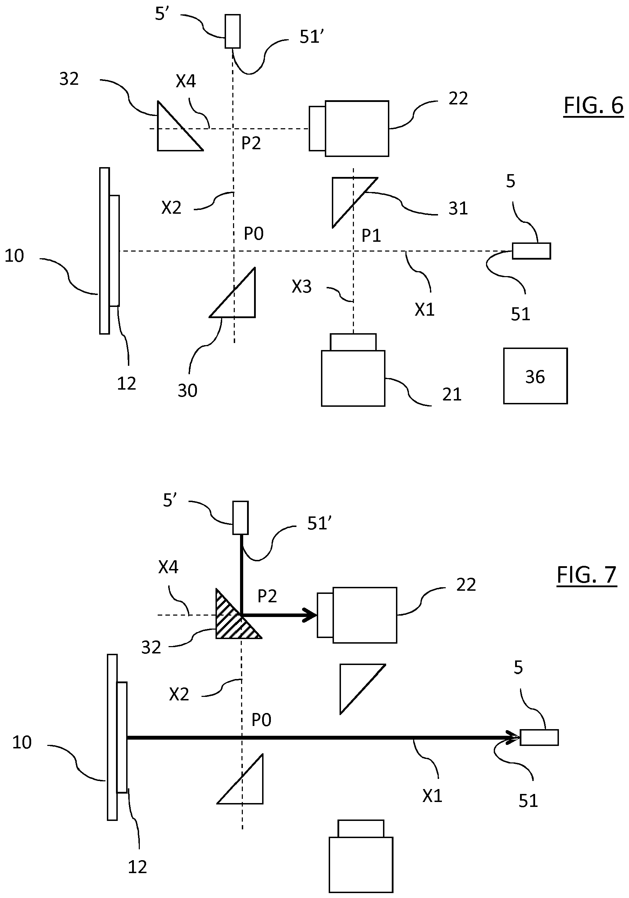

En référence à la figure 6, le guide d'images 5 (respectivement 5') comporte une ouverture proximale 51 (respectivement 51') raccordée à la partie proximale E1 de l'endoscope E et opposée à l'ouverture distale 52 (respectivement 52').

Le générateur 1 de lumière structurée comprend une source 10 de lumière blanche monochromatique, cohérente ou incohérente, et une matrice 12 de micro-miroirs.

La matrice 12 de micro-miroirs est un système électromécanique usuellement utilisé dans les vidéoprojecteurs comprenant un grand nombre de micro-miroirs et permettant la projection d'une image par réflexion de la lumière sur chacun des micro-miroirs. Chaque micro-miroir de la matrice 12 peut prendre deux positions : une position dans laquelle il réfléchit la lumière émise par la source 10 sur un chemin optique et une position dans laquelle il réfléchit la lumière émise par la source vers une surface absorbante. De la sorte, la matrice de micro-miroirs permet de générer un motif de lumière structurée à partir de la lumière émise par la source, le motif dépendant des positions respectives des micro-miroirs. Par ailleurs la modification du motif sur un objet à observer peut être effectuée sans modification du réglage optique de conjugaison de la matrice de micro-miroir et des guides d'images. Elle permet également d'obtenir une très bonne résolution ainsi qu'une grande flexibilité pour générer des codages complexes de la lumière structurée, par exemple dynamiquement (à une fréquence pouvant être supérieure à 100 Hz).

Le générateur 1 peut comprendre, à la place d'une matrice de micro-miroirs 12, un composant d'optique diffractive (optique passive) ou par un réseau de cristaux liquides (piloté activement).

De manière générale, le générateur 1 est adapté pour émettre une pluralité de rayons lumineux dans la direction principale d'un premier axe X1. Une ou plusieurs lentilles (non-illustrées) peuvent être placées à cet effet en sortie de la matrice de micro-miroirs.

Dans la partie proximale E1, l'ouverture proximale 51 du premier guide d'images 5 est centrée sur le premier axe optique X1, en regard de la matrice 12. L'ouverture proximale 51' du deuxième guide d'images 5' est par ailleurs centrée sur un deuxième axe optique X2 croisant le premier axe optique X1 en un point P0. Dans le mode de réalisation illustré, les premiers et deuxièmes axes X1 et X2 sont orthogonaux.

L'aiguilleur optique comprend trois miroirs : un miroir primaire 30 utilisé en voie montante, et deux miroirs secondaires 31, 32 utilisés en voie descendante.

Chaque miroir 30, 31, 32 est monté mobile en translation : chaque miroir est par exemple monté sur un support adapté pour coulisser sur un rail rectiligne parallèle à un axe de déplacement, entre deux positions extrémales. Le déplacement des miroirs est de préférence réalisé au moyen d'actionneur électromagnétique bistable. Les avantages d'un tel actionnement électromagnétique bistable sont un faible temps de basculement et une grande précision de positionnement.

Chaque miroir 30, 31, 32 comporte une surface réfléchissante d'aire suffisante pour réfléchir une image structurée en provenance directe de la matrice 12 (voie montante), ou indirecte, après réflexion sur l'objet à observer (voie descendante). La surface réfléchissante de chaque miroir est par exemple orientée à 45° par rapport à l'axe de déplacement correspondant.

Le miroir primaire 30 est d'une part disposé entre la matrice 12 et l'extrémité proximale 51 du premier guide d'images 5, de façon à ce que son axe de déplacement coïncide avec le deuxième axe optique X2.

Le miroir primaire 30 est disposé de sorte qu'il existe au moins une position dite « active » du miroir primaire 30 le long de son axe de déplacement dans laquelle sa surface réfléchissante coupe le premier axe optique X1 et réfléchit l'intégralité d'une image structurée en provenance directe de la matrice 12.

Dans une position active dite « centrée » du miroir primaire 30, le point d'intersection P0 entre les premier et deuxième axes optiques X1 et X2 est inclus dans le plan de la surface réfléchissante de ce miroir primaire 30.

Le miroir primaire 30 est en outre disposé de sorte qu'il existe au moins une position du miroir primaire 30 le long de son axe de déplacement dans laquelle sa surface réfléchissante ne réfléchit pas d'images structurées en provenance directe de la matrice 12.

Le système d'acquisition 2 comprend une première caméra 21 et une deuxième caméra 22.

Chaque caméra 21, 22 comporte un objectif optique adapté pour recevoir une image structurée incidente.

La première caméra 21 est disposée de façon à ce que l'axe X3 de son objectif coupe le premier axe optique X1 en un point P1 localisé entre le point P0 et l'ouverture proximale du premier guide d'images 5. Dans le mode de réalisation illustré en figure 5, l'axe X3 de l'objectif de la première caméra 21 est orthogonal au premier axe optique X1.

Le premier miroir secondaire 31 est disposé en regard de la première caméra 21 : l'axe de déplacement de ce premier miroir secondaire 31 coïncide avec l'axe de l'objectif de la première caméra 21.

Le premier miroir secondaire 31 est disposé de sorte qu'il existe au moins une position dite « active » du premier miroir secondaire 31 le long de son axe de déplacement dans laquelle sa surface réfléchissante coupe le premier axe optique X1 et réfléchit l'intégralité d'une image structurée en provenance du premier guide d'images 5 utilisé en voie descendante.

Dans une position active particulière dite « centrée » du premier miroir secondaire 31, le point P1 d'intersection entre l'axe optique X1 et l'axe de déplacement X3 du premier miroir secondaire 31 est inclus dans le plan de la surface réfléchissante du premier miroir secondaire 31.

Le premier miroir secondaire 31 est en outre disposé de sorte qu'il existe au moins une position dite « passive » du premier miroir secondaire 31 le long de son axe de déplacement dans laquelle sa surface réfléchissante ne réfléchit pas d'images structurée en provenance de l'ouverture proximale 51 du premier guide d'images 5.

La deuxième caméra 22 est disposée de façon à ce que l'axe X4 de son objectif coupe le deuxième axe optique X2 en un point P2 localisé entre le point P0 et l'extrémité proximale 51' du deuxième guide d'images 5'. Dans le mode de réalisation illustré en figure 5, l'axe X4 de l'objectif de la deuxième caméra 22 est orthogonal au deuxième axe optique X2.

Le deuxième miroir secondaire 32 est disposé en regard de la deuxième caméra 22 : l'axe de déplacement de ce deuxième miroir secondaire 32 coïncide avec l'axe X4 de l'objectif de la deuxième caméra 22.

Le deuxième miroir secondaire 32 est disposé de sorte qu'il existe au moins une position dite « active » du deuxième miroir secondaire 32 le long de son axe de déplacement X4 dans laquelle sa surface réfléchissante coupe le premier axe optique X1 et réfléchit l'intégralité d'une image structurée en provenance de l'ouverture proximale 51' du deuxième guide d'images 5' utilisé en voie descendante.

Dans une position active particulière dite « centrée » du deuxième miroir secondaire 32, le point P2 d'intersection entre le premier axe optique X1 et l'axe de déplacement du deuxième miroir secondaire 32 est inclus dans le plan de la surface réfléchissante du deuxième miroir secondaire 32.

Le deuxième miroir secondaire 32 est en outre disposé de sorte qu'il existe au moins une position dite « passive » du deuxième miroir secondaire 32 le long de son axe de déplacement dans laquelle sa surface réfléchissante ne réfléchit pas d'images structurée en provenance de l'ouverture proximale 51' du deuxième guide d'images 5'.

Des lentilles optiques peuvent par ailleurs être agencées dans l'aiguilleur optique pour conjuguer optiquement deux à deux les éléments suivants: la matrice 12 et l'ouverture proximale 51, la matrice 12 et l'ouverture proximale 51', la caméra 21 et l'ouverture proximale 51, et la caméra 22 et l'ouverture proximale 51' (ces lentilles ne sont pas illustrées sur les figures pour plus de simplicité).

L'aiguilleur optique 3 comporte également une unité de commande 36 adaptée pour contrôler le déplacement des miroirs 30, 31, 32 le long de leurs axes respectifs. L'unité de commande est par ailleurs adaptée pour activer ou désactiver le générateur 1 et la source lumineuse secondaire 8.

L'unité de commande 36 comprend par exemple un ou plusieurs processeurs et une interface de communication avec chaque élément qu'elle commande. Par exemple, L'unité de commande 36 est connectée avec chacun de ces éléments par une liaison filaire (non-illustrée sur les figures pour une meilleure lisibilité).

Chaque processeur de l'unité de commande 36 est configuré pour mettre en oeuvre des instructions de code générant des commandes qui sont communiquées aux éléments à commander via l'interface de communication.

La désactivation du générateur 1 par l'unité de commande 36 peut comprendre une mise hors tension de la source 10 et/ou une configuration de chaque micro-miroir de la matrice 12 dans sa position où il réfléchit la lumière émise par la source 10 vers une surface absorbante. Par opposition, le générateur 1 est considéré comme activé lorsqu'il transmet de la lumière structurée à l'aiguilleur optique 3.

La désactivation de la source lumineuse secondaire 8 peut en outre être réalisée par mise hors tension de cette source 8. Par opposition, la source 8 est considérée comme activée lorsque qu'elle transmet de la lumière dans la fibre optique 7.

### Pilotage des miroirs primaire et secondaires

Pour basculer l'endoscope E dans son premier mode de fonctionnement (vision active voie gauche), l'unité de commande 36 déplace :
- le miroir primaire 30 dans une position passive,
- le premier miroir secondaire 31 dans une position passive, et
- le deuxième miroir secondaire 32 dans une position active.
L'unité de commande 36 active par ailleurs le générateur 1 et désactive la source lumineuse secondaire 8.

Dans le premier mode de fonctionnement, illustré en figure 7, la lumière émise par la source lumineuse 10 est structurée par la matrice 12, longe le premier axe optique X1, atteint l'ouverture proximale 51 du premier guide d'images 5, est acheminée par le premier guide 5 en voie montante jusqu'à l'embout distal 6, est réfléchie par l'objet à observer, pénètre à nouveau dans l'embout distal 6 puis dans le deuxième guide d'images 5' en voie descendante jusqu'à atteindre son extrémité proximale 51', longe l'axe X2, est réfléchie par le deuxième miroir secondaire 32 en position active sur l'axe X4, et atteint enfin l'objectif de la deuxième caméra 22.

Par ailleurs, pour basculer l'endoscope dans le deuxième mode de fonctionnement (vision active voie droite), l'unité de commande 36 déplace :
- le miroir primaire 30 dans une position active,
- le premier miroir secondaire 31 dans une position active, et
- le deuxième miroir secondaire 32 dans une position passive.
L'unité de commande 36 active par ailleurs le générateur 1 et désactive la source lumineuse secondaire 8, si ce n'est pas déjà le cas.

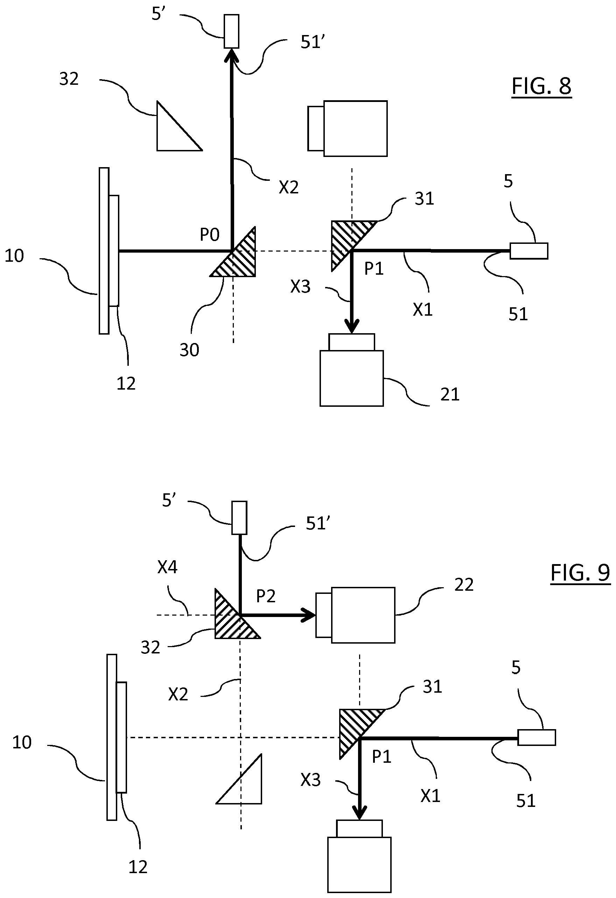

Dans le deuxième mode de fonctionnement, illustré en figure 8, la lumière émise par la source lumineuse 10 est structurée par la matrice 12, longe le premier axe optique X1, est réfléchie par le miroir primaire 30, est redirigée le long du deuxième axe optique X2, atteint l'ouverture proximale 51' du deuxième guide d'images 5', est acheminée en voie montante jusqu'à l'embout distal par le guide 5', est réfléchie par l'objet à observer, pénètre à nouveau dans l'embout distal puis dans le premier guide d'images 5, est acheminée en voie descendante jusqu'à atteindre son l'ouverture proximale 51, longe l'axe X1, est réfléchie par le premier miroir secondaire 31 en position active sur l'axe X3, et atteint enfin l'objectif de la première caméra 21.

En outre, pour basculer l'endoscope dans le troisième mode de fonctionnement (stéréovision passive), l'unité de commande déplace chacun des deux miroirs secondaires 31, 32 dans une position active. L'unité de commande 36 désactive par ailleurs le générateur 1 et active la source lumineuse secondaire 8.

Dans le troisième mode de fonctionnement, illustré en figure 9, de la lumière générée par la source lumineuse secondaire 8 se propage dans la fibre optique 7 jusqu'à l'embout distal 6, sort de l'endoscope par l'embout distal 6 et se réfléchit en une zone de la surface S d'un objet tridimensionnel à observer. La lumière structurée ainsi réfléchie pénètre à nouveau dans l'endoscope par l'embout distal 6, se propage par voie descendante dans les deux guides d'images 5 et 5' non utilisés en voie montante, puis est aiguillée par l'aiguilleur optique 3 vers le système d'acquisition 2. La lumière non structurée en provenance des deux guides d'images 5 et 5', parvient aux deux caméras 21, 22 après réflexion sur les surfaces réfléchissantes respectives des deux miroirs secondaires 31 et 32.

L'endoscope E peut ainsi, au moyen de seulement trois miroirs, être exploité selon les trois modes de fonctionnements décrits, chaque mode permettant de collecter des informations visuelles sur l'objet observé.

Pour basculer dans chacun de ces modes de fonctionnement (par exemple, de l'un à l'autre) les miroirs 30, 31, 32 peuvent être déplacés de façon simultanée de manière à raccourcir le temps de bascule et/ou éviter des configurations non désirées de l'aiguilleur optique 3, telles qu'avoir le miroir primaire 30 et le premier miroir secondaire 31 simultanément actifs.

Des lentilles fixes et diaphragmes peuvent être agencés le long des axes X1 et X2 de façon à focaliser les rayons lumineux sur une zone précise. L'image de 12 se forme en 51 (et non avant ou après 51) grâce aux lentilles. L'image de 12 se forme en 51' également grâce aux lentilles. De même l'image de 51' se forme sur le capteur de 22 grâce aux lentilles et l'image de 51 se forme sur le capteur de 21 grâce aux lentilles. L'ajout de diaphragmes avec les lentilles permet de régler et d'améliorer la netteté et/ou le contraste des images.

L'endoscope peut comporter également une unité de traitement d'images (non-illustrée) adaptée pour traiter les images acquises par le système d'acquisition optique 2 et reconstruire l'enveloppe tridimensionnelle de l'objet observé à partir des images acquises.

L'endoscope E décrit peut être utilisé pour tout type d'inspection d'organes, par exemple pour réaliser une coloscopie ou une laparoscopie, ainsi pour l'inspection de pièces en environnement contraint, par exemple de l'inspection tubulaire, et en milieu industriel.

Par ailleurs, les différentes parties illustrées sur les figures 4 à 9 peuvent être réalisées dans un dispositif d'imagerie autre qu'un endoscope, par exemple un dispositif de vérification d'un mécanisme d'horlogerie.

### Deuxième mode de réalisation de partie distale

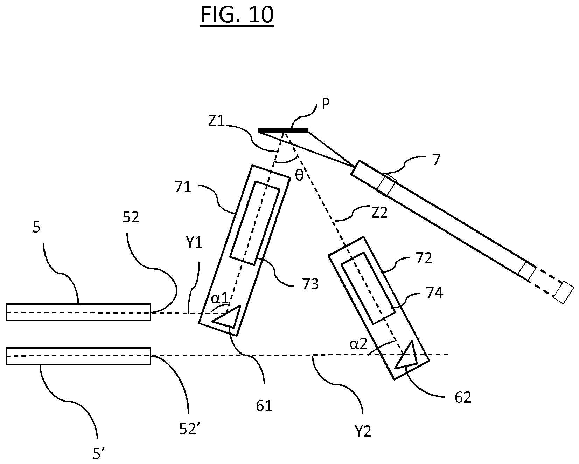

Un deuxième mode de réalisation de partie distale pour un dispositif d'imagerie va maintenant être détaillé en relation avec la figure 10.

Les deux guides d'images 5 et 5' comprennent chacun un corps creux rigide, par exemple de forme cylindrique, et un agencement de lentilles et/ou de miroirs agencés dans ce corps creux.

Dans le dispositif E selon ce deuxième mode de réalisation, comprend en outre deux bras 71, 72.

Chaque bras est configuré pour diffuser la lumière acheminée par l'un des guides d'images et rediriger vers ce guide d'image 5, 5' la lumière réfléchie par l'objet O.

Chaque bras constitue donc un embout distal pour le dispositif d'imagerie E selon ce deuxième mode de réalisation.

Les deux bras sont mobiles l'un par rapport à l'autre de sorte à former un angle de réflexion variable avec l'objet à observer en fonction de la distance de l'objet par rapport aux deux bras.

Plus précisément, le premier bras 71 comprend un corps creux rigide présentant deux ouvertures : une ouverture proximale et une ouverture distale.

L'ouverture proximale du premier bras 71 est en communication optique avec l'ouverture distale du premier guide d'images 5, centrée sur l'axe Y1.

Le corps du premier bras 71 contient le premier élément optique réfléchissant 61, qui est fixe par rapport au corps. Le corps du premier bras 71 peut également comprendre une interface optique telle qu'un agencement de lentilles 73.

Le corps du premier bras 71 est adapté pour acheminer de la lumière entre son ouverture proximale et une surface réfléchissante du premier élément réfléchissant 61 le long de l'axe Y1, et acheminer de la lumière entre cette surface réfléchissante et l'ouverture distale du corps, le long de l'axe optique Z1.

Le corps du premier bras 71 est monté mobile par rapport au corps du premier guide d'image 5, par exemple au moyen d'une liaison articulée (non illustrée), telle qu'une liaison pivot.

L'axe optique Z1 forme de la sorte un α1 de réflexion variable par rapport à l'axe optique Y1, en fonction de la position du bras en rotation par rapport au guide d'images 5.

Similairement, le deuxième bras 72 comprend un corps creux rigide présentant deux ouvertures : une ouverture proximale et une ouverture distale.

L'ouverture proximale du deuxième bras 72 est en communication optique avec l'ouverture distale du deuxième guide d'images 5', centrée sur l'axe Y2.

Le corps du deuxième bras 72 contient le premier élément optique réfléchissant 62, qui est fixe par rapport au corps. Le corps du deuxième bras 72 peut également comprendre une interface optique telle qu'un agencement de lentilles 74.

Le corps du deuxième bras 72 est adapté pour acheminer de la lumière entre son ouverture proximale et une surface réfléchissante du premier élément réfléchissant 62 le long de l'axe Y2, et acheminer de la lumière entre cette surface réfléchissante et l'ouverture distale du corps, le long de l'axe optique Z2.

Le corps du deuxième bras 72 est monté mobile par rapport au corps du deuxième guide d'images 5', par exemple au moyen d'une liaison articulée (non illustrée), telle qu'une liaison pivot.

L'axe optique Z2 forme de la sorte un angle α2 de valeur variable par rapport à l'axe optique Y2, en fonction de la position du deuxième bras mobile 72 par rapport au deuxième guide d'images 5'.

Pour différents couples d'anges (α1, a2), les axes optiques Z1 et Z2 des deux bras se rencontrent en un point P à distance variable par rapport aux bras, avec un angle de réflexion θ de lumière sur une surface contenant le point P également variable.

Chaque bras peut être déplacé au moyen d'un actionneur dédié (non-représenté sur les figures), commandé par l'unité de commande 36.

Le dispositif d'imagerie selon le deuxième mode de réalisation comprend également la fibre optique 7 prévue pour acheminer de la lumière structurée ou non, émise par la source secondaire 8. Cette fibre optique est ici représentée libre par rapport aux deux bras 71 et 72 et par rapport aux deux guides d'images 5 et 5'.

### Deuxième mode de réalisation de partie proximale

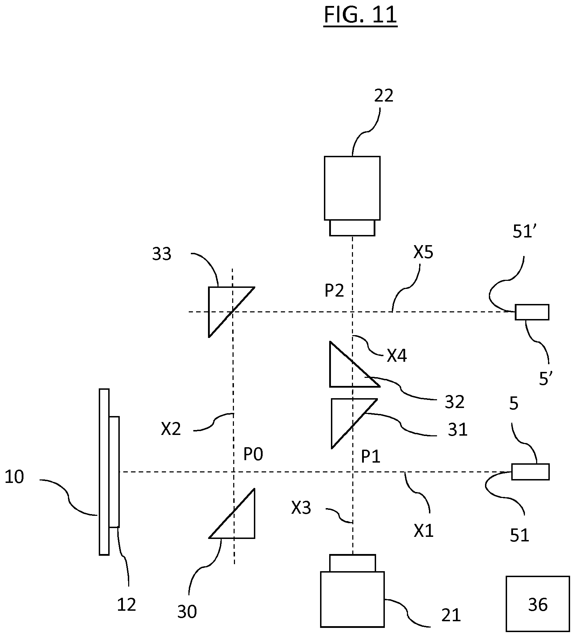

Un deuxième mode de réalisation de partie proximale pour un dispositif d'imagerie va maintenant être détaillé en relation avec les figures 11 à 14.

En référence à la **figure 11****,** le dispositif d'imagerie E selon ce deuxième mode de réalisation reprend les éléments suivants : la source 10, la matrice 12, le miroir primaire 30, les miroirs secondaires 31, 32, les deux caméras 21, 22 déjà décrits.

Tout comme dans le précédent mode de réalisation illustré, l'ouverture proximale 51 du premier guide d'images 5 est centrée sur le premier axe optique X1, en regard de la matrice 12, et le miroir primaire 30 est agencé de façon à croiser le premier axe optique X1 en un point P0. Dans le mode de réalisation illustré, les premiers et deuxièmes axes X1 et X2 sont orthogonaux.

Ce mode de réalisation diffère toutefois de celui illustré sur les figures 6 à 9 notamment par le fait qu'il comprend un miroir fixe supplémentaire 33.

Le miroir fixe 33 est agencé sur le chemin optique entre le générateur de lumière structurée et l'un des deux guides d'images, ici le guide 5'.

L'ouverture proximale 51' du guide 5' est centrée sur un axe optique X5, en regard du miroir fixe 33, différent de l'axe optique X2 et orthogonal à celui-ci.

Le miroir fixe supplémentaire 33 est disposé de sorte que sa surface réfléchissante coupe les axes optiques X2 et X5, de façon à réfléchir l'intégralité d'une image structurée en provenance directe de la matrice 12.

Les deux axes optiques X1 et X5 sur lesquels sont centrés les ouvertures proximales des deux guides optiques 5, 5' sont parallèles, et tous deux orthogonaux à l'axe optique X2.

Dans ce deuxième mode de réalisation, les deux ouvertures proximales des deux guides d'images peuvent ainsi être positionnées proches l'un de l'autre, ce qui réduit l'encombrement général du dispositif d'imagerie E. De plus, placer les deux guides d'images selon une même orientation permet également de réaliser plus simplement la partie distale (notamment lorsqu'elle est rigide).

Par ailleurs, dans ce deuxième mode de réalisation, le miroir secondaire 32 est positionné de façon à couper l'axe optique X5 entre le miroir fixe 33 et l'ouverture 51' du guide d'image 5', dans sa position active.

Les axes optiques X3 et X4 des deux caméras 21 et 22 (et de déplacement des miroirs secondaires 31 et 32) sont confondus.

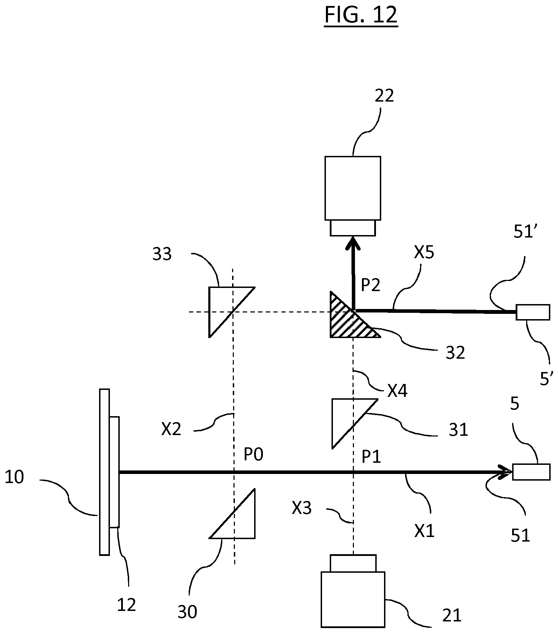

Pour configurer l'aiguilleur optique 3 du dispositif E dans le premier mode de fonctionnement, les miroirs 30, 31 et 32 sont déplacés conformément à la **figure 12** **:**
- le miroir primaire 30 dans une position passive,
- le premier miroir secondaire 31 dans une position passive, et
- le deuxième miroir secondaire 32 dans une position active.

La lumière structurée émise par la matrice 12 longe l'axe optique X1 jusqu'à l'ouverture proximale 51. Le premier guide 5 transporte la lumière structure jusqu'à l'ouverture distale 51. La lumière pénètre dans le premier bras 71, est réfléchie par le premier élément réfléchissant 61 et ressort du premier bras par son ouverture distale en regard de l'objet O à observer. Après réflexion sur cet objet O, la lumière pénètre dans le deuxième bras 72, se réfléchit sur le deuxième élément réfléchissant 62, pénètre dans le deuxième guide d'images 5' par son ouverture distale 52'. Ensuite, cette lumière réfléchie ressort du guide 5' par son ouverture proximale 51', longe l'axe optique X5, est réfléchie par le miroir 32 et est acquise par la caméra 22.

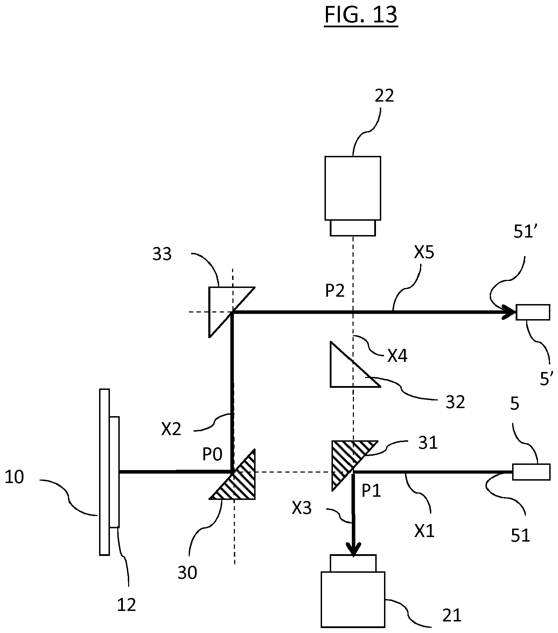

Pour configurer l'aiguilleur optique 3 du dispositif E dans le deuxième mode de fonctionnement, les miroirs 30, 31 et 32 sont déplacés conformément à la figure 13 :
- le miroir primaire 30 dans une position active,
- le premier miroir secondaire 31 dans une position active, et
- le deuxième miroir secondaire 32 dans une position passive.

La lumière structurée émise par la matrice 12 longe l'axe optique X1, est réfléchie par le miroir primaire 50 puis par le miroir fixe 53, puis parvient jusqu'à l'ouverture proximale 51'. Le deuxième guide 5' transporte la lumière structurée jusqu'à l'ouverture distale 51'. La lumière pénètre dans le deuxième bras 73, est réfléchie par le deuxième élément réfléchissant 62 et ressort du deuxième bras par son ouverture distale en regard de l'objet O à observer. Après réflexion sur cet objet O, la lumière pénètre dans le premier bras 71, se réfléchit sur le premier élément réfléchissant 61, pénètre dans le deuxième guide d'images 5 par son ouverture distale 52'. Ensuite, cette lumière réfléchie ressort du guide 5 par son ouverture proximale 51, longe l'axe optique X1, est réfléchie par le miroir 31 et est acquise par la caméra 21.

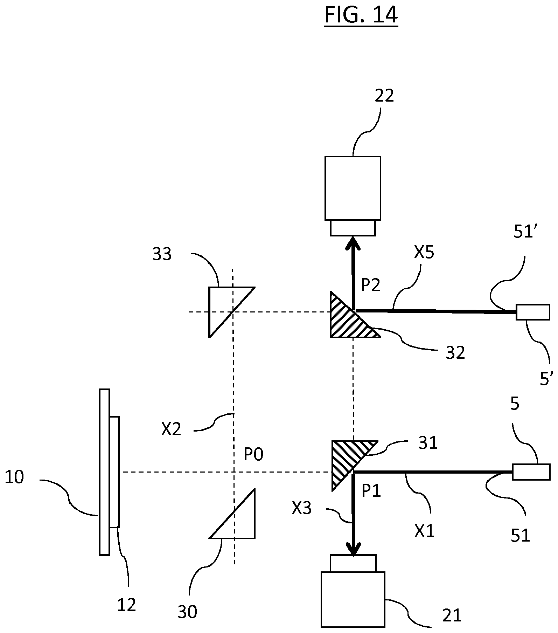

En outre, pour basculer le dispositif d'imagerie dans le troisième mode de fonctionnement (stéréovision passive), l'unité de commande 36 déplace chacun des deux miroirs secondaires 31, 32 dans une position active conformément à la **figure 14****.** L'unité de commande 36 désactive par ailleurs le générateur 1 et active la source lumineuse secondaire 8.

La lumière générée par la source lumineuse éclairant l'objet O est captée par chacun des deux bras 71, 72, guidée par les deux guides 5 et 5' alors utilisés comme voies descendantes. Cette lumière parvient aux caméras 21 et 22 après réflexion par les miroirs secondaires 31 et 32.

D'autres modes de réalisations (non-illustrés) sont également possibles. Une liste non-limitative de variantes portant sur une ou plusieurs caractéristiques du dispositif d'imagerie E est la suivante.
- Les premiers et deuxièmes axes optiques X1, X2 peuvent être sécants sans être orthogonaux.
- Les axes optiques X1 et X3 peuvent être sécants sans être orthogonaux.
- Les axes optiques X2 et X4 peuvent être sécants sans être orthogonaux.
- Les miroirs peuvent être mobile en rotation et/ou être remplacés par d'autres éléments optiques adaptés pour rediriger un rayon de lumière incident selon au moins deux directions de sortie alternatives.
- Les miroirs peuvent être déplacés de façon non simultanée.
- Les miroirs peuvent éventuellement être remplacés par des cubes séparateurs (« beams splitters » en anglais) ou par des lames séparatrices non actionnés.
- D'autres éléments optiques (lentilles, miroirs, etc.) peuvent être incorporés à l'aiguilleur optique de façon à élaborer des chemins optiques plus complexes entre le générateur 1, le système d'acquisition 3 et les guides d'images 5 et 5'.
- le dispositif d'imagerie peut comporter plus de deux guides d'images structurées, au moins un des guides étant sollicité en voie montante et au moins un guide étant sollicité en parallèle en voie descendante,
- La fibre optique et les guides d'images structurées peuvent être raccordés à la même ouverture O.
- L'ouverture O peut être latérale, comme représentée sur les figures, ou être ménagée en l'extrémité distale de l'embout 6.
- Le nombre de voies montantes ou descendantes peut être augmenté pour dépasser la valeur de 2, chaque voie correspondant à un angle de vue respective de l'objet à observer, de façon à enrichir les données d'images acquises de cet objet.
- Le nombre de fibres optiques d'éclairage peut être augmenté. Cela permet d'éclairer en mode passif sous différents angles et donc de modifier les zones spéculaires problématiques.
- les guides d'images peuvent être insérés dans les canaux opérateurs d'un coloscope conventionnel pour permettre la mesure 3D tout en conservant le mode de fonctionnement habituel du coloscope conventionnel.

## Revendications

1. Dispositif (E) d'imagerie comprenant :
- au moins un générateur (1) de lumière structurée,
- un premier guide d'images (5, 5') d'émission pour acheminer par voie montante la lumière structurée depuis le générateur (1) vers un objet à observer,
- un deuxième guide d'images (5', 5) de retour pour acheminer par voie descendante la lumière réfléchie par ledit objet à observer, jusqu'à un système d'acquisition (2) de ladite lumière réfléchie,
le dispositif (E) étant **caractérisé en ce que** chacun (5, 5') des deux guides d'images est apte à acheminer la lumière structurée par voie montante et la lumière réfléchie par voie descendante, et **en ce que** le dispositif (E) comprend un aiguilleur optique (3) configurable:
- dans un premier mode de fonctionnement dans lequel il dirige sélectivement la lumière structurée issue du générateur (1) vers le premier guide d'images, par voie montante, et dirige dans le même temps sélectivement la lumière réfléchie par l'objet depuis le deuxième guide d'images, par voie descendante, jusqu'au système d'acquisition (2),
- dans un deuxième mode de fonctionnement dans lequel il dirige sélectivement la lumière structurée issue du générateur (1) vers le deuxième guide d'images, par voie montante, et diriger dans le même temps sélectivement la lumière réfléchie par l'objet depuis le premier guide d'images, par voie descendante, jusqu'au système d'acquisition (2), et
- dans un troisième mode de fonctionnement dans lequel il dirige simultanément de la lumière en provenance des deux guides d'images (5, 5') par voie descendante jusqu'au système d'acquisition (2).

2. Dispositif (E) selon la revendication 1, comprenant en outre une source lumineuse secondaire (8) adaptée pour éclairer l'objet.

3. Dispositif (E) selon la revendication 2, comprenant en outre une fibre optique (7) pour acheminer de la lumière émise par la source secondaire (8) vers l'objet.

4. Dispositif (E) selon l'une des revendications précédentes, comprenant en outre un embout distal (6) configuré pour diffuser de la lumière acheminée par chacun des deux guides d'images (5, 5') sur l'objet à observer et rediriger vers chacun des deux guides d'images (5, 5') de la lumière réfléchie par ledit objet.

5. Dispositif (E) selon l'une des revendications précédentes, comprenant en outre deux bras distaux, chaque bras distal étant configuré pour diffuser la lumière acheminée par l'un des guides d'images et rediriger vers ce guide d'image (5, 5') la lumière réfléchie par ledit objet, les deux bras étant mobiles l'un par rapport à l'autre de sorte à former un angle de réflexion variable avec l'objet à observer en fonction de la distance de l'objet par rapport aux deux bras.

6. Dispositif (E) selon l'une des revendications précédentes, dans lequel chaque guide d'images (5, 5') comprend un faisceau de fibres optiques.

7. Dispositif (E) selon l'une des revendications précédentes, dans lequel chaque guide d'images (5, 5') comprend un agencement de miroirs et/ou de lentilles.

8. Dispositif (E) selon l'une des revendications précédentes, dans lequel le dispositif est un endoscope.

9. Dispositif (E) selon l'une des revendications précédentes, dans lequel l'aiguilleur optique (3) comprend un miroir primaire (30) disposé entre le générateur (1) et les deux guides d'images (5, 5'), le miroir primaire étant adapté pour permettre une redirection sélective de lumière structurée en provenance du générateur (1) vers un premier axe optique (X1) menant au premier guide d'images (5) ou vers un deuxième axe (X2) optique menant au deuxième guide d'images (5').

10. Dispositif (E) selon la revendication précédente, dans lequel le générateur (1) est configuré pour émettre de la lumière structurée selon le premier axe (X1) menant au premier guide d'images (5), et le miroir primaire (30) est mobile entre :
- une position active dans laquelle le miroir primaire (30) coupe le premier axe (X1) entre les générateur (1) et le premier guide d'images (5) et réoriente vers le deuxième guide d'images (5') la lumière structurée en provenance des générateur (1) selon le deuxième axe (X2), et
- une position passive dans laquelle le miroir primaire (30) ne coupe pas le premier axe (X1).

11. Dispositif (E) selon la revendication précédente, comprenant en outre un autre miroir (33) agencé pour rediriger la lumière réfléchie par le miroir primaire (31), lorsqu'il est positionné dans sa position active, sur un autre axe (X5) parallèle au premier axe (X1), le premier guide présentant une ouverture proximale alignée sur le premier axe (X1), et le deuxième guide présentant une ouverture proximale alignée sur l'autre axe (X5).

12. Dispositif (E) selon l'une des revendications 10 et 11, dans lequel :
- l'aiguilleur optique (3) comprend un premier miroir secondaire (31) un deuxième miroir secondaire (32),
- le premier miroir secondaire (31) est mobile entre
o une position active dans laquelle le premier miroir secondaire (31) coupe le premier axe (X1) entre le générateur (1) et le premier guide d'images (5), et réoriente vers une caméra (21) de la lumière émanant du premier guide d'images (5), et
o une position passive dans laquelle le premier miroir secondaire (31) ne coupe pas le premier axe (X1),
- le deuxième miroir secondaire (32) est mobile entre
o une position active dans laquelle le deuxième miroir secondaire (32) coupe le deuxième axe (X2) entre le générateur (1) et le miroir primaire (30), et réoriente vers le système d'acquisition (2) de la lumière émanant du deuxième guide d'images (5'), et
o une position passive dans laquelle le premier miroir secondaire (31) ne coupe pas le premier axe (X1).

13. Dispositif (E) selon la revendication précédente, dans lequel l'aiguilleur optique (3) comprend en outre une unité de commande (36) adaptée pour :
- dans le premier mode de fonctionnement, positionner le miroir primaire (30) et le premier miroir secondaire (31) dans une position passive et le deuxième miroir secondaire (32) dans une position active, et
- dans le deuxième mode de fonctionnement, positionner le miroir primaire (30) et le premier miroir secondaire (31) dans une position active et le deuxième miroir secondaire (32) dans une position passive,
- dans le troisième mode de fonctionnement, positionner les miroirs secondaires (31, 32) dans une position active.

14. Dispositif (E) selon l'une des revendications 12 à 13, dans lequel le miroir primaire (30) et les miroirs secondaires (31, 32) sont chacun mobiles en translation au moyen d'au moins un actionneur électromagnétique bistable.

15. Dispositif (E) selon l'une des revendications précédentes, dans lequel le système d'acquisition (2) comprend deux caméras (21, 22), chaque caméra étant agencée pour recevoir de la lumière en provenance d'un des deux guides d'images (5, 5').

16. Procédé d'imagerie comprenant les étapes de :
- générer de la lumière structurée,
- acheminer dans un premier guide d'images par voie montante la lumière structurée générée vers un objet à observer,
- acheminer dans un deuxième guide d'images par voie descendante la lumière réfléchie par ledit objet à observer jusqu'à un système d'acquisition (2) de ladite lumière réfléchie,
- acquérir la lumière réfléchie au moyen du système d'acquisition,
le procédé étant **caractérisé par** les étapes suivantes, chacun des deux guides d'images étant apte à acheminer la lumière structurée par voie montante et la lumière réfléchie par voie descendante:
- diriger sélectivement la lumière structurée générée vers le premier guide d'images, par voie montante, et diriger dans le même temps sélectivement la lumière réfléchie par l'objet depuis le deuxième guide d'images, par voie descendante, jusqu'au système d'acquisition (2),
- diriger sélectivement la lumière structurée générée vers le deuxième guide d'images, par voie montante, et diriger dans le même temps sélectivement la lumière réfléchie par l'objet depuis le premier guide d'images, par voie descendante, jusqu'au système d'acquisition (2), et
- diriger simultanément de la lumière en provenance des deux guides d'images (5, 5') par voie descendante jusqu'au système d'acquisition (2).

## Patentansprüche

1. Bildgebungsvorrichtung (E), umfassend:
- mindestens einen Erzeuger (1) von strukturiertem Licht,
- einen ersten Sende-Bildleiter (5, 5'), um das strukturierte Licht in Aufwärtsrichtung vom Erzeuger (1) zu einem zu beobachtenden Objekt zu leiten,
- einen zweiten Rück-Bildleiter (5', 5), um das Licht, das von dem zu beobachtenden Objekt reflektiert wird, in Abwärtsrichtung bis zu einem System zum Erfassen (2) des reflektierten Lichts zu leiten,
wobei die Vorrichtung (E) **dadurch gekennzeichnet ist, dass** jeder (5, 5') der zwei Bildleiter das strukturierte Licht in Aufwärtsrichtung und das reflektierte Licht in Abwärtsrichtung leiten kann, und dadurch, dass die Vorrichtung (E) eine optische Weiche (3) umfasst, die konfigurierbar ist:
- in einem ersten Betriebsmodus, in dem sie das aus dem Erzeuger (1) stammende strukturierte Licht selektiv in Aufwärtsrichtung zum ersten Bildleiter lenkt, und zur gleichen Zeit das vom Objekt reflektierte Licht selektiv in Abwärtsrichtung vom zweiten Bildleiter bis zum Erfassungssystem (2) lenkt,
- in einem zweiten Betriebsmodus, in dem sie das aus dem Erzeuger (1) stammende strukturierte Licht selektiv in Aufwärtsrichtung zum zweiten Bildleiter lenkt, und zur gleichen Zeit das vom Objekt reflektierte Licht selektiv in Abwärtsrichtung vom ersten Bildleiter bis zum Erfassungssystem (2) lenkt, und
- in einem dritten Betriebsmodus, in dem sie Licht aus den zwei Bildleitern (5, 5') gleichzeitig in Abwärtsrichtung bis zum Erfassungssystem (2) lenkt.

2. Vorrichtung (E) nach Anspruch 1, weiter eine sekundäre Lichtquelle (8) umfassend, die dafür geeignet ist, das Objekt zu beleuchten.

3. Vorrichtung (E) nach Anspruch 2, weiter eine optische Faser (7) umfassend, um Licht, das von der sekundären Quelle (8) ausgesendet wird, zum Objekt zu leiten.

4. Vorrichtung (E) nach einem der vorstehenden Ansprüche, weiter ein distales Endstück (6) umfassend, das dafür konfiguriert ist, Licht, das von jedem der zwei Bildleiter (5, 5') geleitet wird, auf dem zu beobachtenden Objekt zu verteilen, und Licht, das von dem Objekt reflektiert wird, zu jedem der zwei Bildleiter (5, 5') zurückzulenken.

5. Vorrichtung (E) nach einem der vorstehenden Ansprüche, weiter zwei distale Schenkel umfassend, wobei jeder distale Schenkel dafür konfiguriert ist, das Licht, das von einem der Bildleiter geleitet wird, zu verteilen, und das Licht, das vom Objekt reflektiert wird, zu diesem Bildleiter (5, 5') zurückzulenken, wobei die zwei Schenkel zueinander beweglich sind, um abhängig vom Abstand des Objekts in Bezug auf die zwei Schenkel einen veränderlichen Reflexionswinkel zu dem zu beobachtenden Objekt zu bilden.

6. Vorrichtung (E) nach einem der vorstehenden Ansprüche, wobei jeder Bildleiter (5, 5') Bündel optischer Fasern umfasst.

7. Vorrichtung (E) nach einem der vorstehenden Ansprüche, wobei jeder Bildleiter (5, 5') eine Anordnung von Spiegeln und/oder von Linsen umfasst.

8. Vorrichtung (E) nach einem der vorstehenden Ansprüche, wobei die Vorrichtung ein Endoskop ist.

9. Vorrichtung (E) nach einem der vorstehenden Ansprüche, wobei die optische Weiche (3) einen Primärspiegel (30) umfasst, der zwischen dem Erzeuger (1) und den zwei Bildleitern (5, 5') angeordnet ist, wobei der Primärspiegel dafür geeignet ist, ein selektives Umlenken von strukturiertem Licht aus dem Erzeuger (1) zu einer ersten optischen Achse (X1), die zum ersten Bildleiter (5) führt, oder zu einer zweiten optischen Achse (X2), die zum zweiten Bildleiter (5') führt, zu ermöglichen.

10. Vorrichtung (E) nach dem vorstehenden Anspruch, wobei der Erzeuger (1) dafür konfiguriert ist, strukturiertes Licht entlang der zum ersten Bildleiter (5) führenden ersten Achse (X1) auszusenden, und der Primärspiegel (30) beweglich ist zwischen:
- einer aktiven Stellung, in der der Primärspiegel (30) die erste Achse (X1) zwischen dem Erzeuger (1) und dem ersten Bildleiter (5) schneidet und das strukturierte Licht aus dem Erzeuger (1) entlang der zweiten Achse (X2) zum zweiten Bildleiter (5') umlenkt, und
- einer passiven Stellung, in der der Primärspiegel (30) die erste Achse (X1) nicht schneidet.

11. Vorrichtung (E) nach dem vorstehenden Anspruch, weiter einen anderen Spiegel (33) umfassend, der dafür eingerichtet ist, das Licht, das vom Primärspiegel (31) reflektiert wird, wenn dieser in seiner aktiven Stellung positioniert ist, zu einer anderen Achse (X5) umzulenken, die zur ersten Achse (X1) parallel ist, wobei der erste Leiter eine proximale Öffnung aufweist, die mit der ersten Achse (X1) fluchtet, und der zweite Leiter eine proximale Öffnung aufweist, die mit der anderen Achse (X5) fluchtet.

12. Vorrichtung (E) nach einem der Ansprüche 10 und 11, wobei:
- die optische Weiche (3) einen ersten Sekundärspiegel (31) und einen zweiten Sekundärspiegel (32) umfasst,
- der erste Sekundärspiegel (31) beweglich ist zwischen
-- einer aktiven Stellung, in der der erste Sekundärspiegel (31) die erste Achse (X1) zwischen dem Erzeuger (1) und dem ersten Bildleiter (5) schneidet und Licht, das aus dem ersten Bildleiter (5) austritt, zu einer Kamera (21) umlenkt, und
-- einer passiven Stellung, in der der erste Sekundärspiegel (31) die erste Achse (X1) nicht schneidet,
- der zweite Sekundärspiegel (32) beweglich ist zwischen
-- einer aktiven Stellung, in der der zweite Sekundärspiegel (32) die zweite Achse (X2) zwischen dem Erzeuger (1) und dem Primärspiegel (30) schneidet und Licht, das aus dem zweiten Bildleiter (5') austritt, zum Erfassungssystem (2) umlenkt, und
-- einer passiven Stellung, in der der erste Sekundärspiegel (31) die erste Achse (X1) nicht schneidet.

13. Vorrichtung (E) nach dem vorstehenden Anspruch, wobei die optische Weiche (3) weiter eine Steuereinheit (36) umfasst, die dafür geeignet ist:
- im ersten Betriebsmodus den Primärspiegel (30) und den ersten Sekundärspiegel (31) in einer passiven Stellung, und den zweiten Sekundärspiegel (32) in einer aktiven Stellung zu positionieren, und
- im zweiten Betriebsmodus den Primärspiegel (30) und den ersten Sekundärspiegel (31) in einer aktiven Stellung, und den zweiten Sekundärspiegel (32) in einer passiven Stellung zu positionieren,
- im dritten Betriebsmodus die Sekundärspiegel (31, 32) in einer aktiven Stellung zu positionieren.

14. Vorrichtung (E) nach einem der Ansprüche 12 bis 13, wobei der Primärspiegel (30) und die Sekundärspiegel (31, 32) jeder mittels mindestens eines bistabilen elektromagnetischen Aktors translationsbeweglich sind.

15. Vorrichtung (E) nach einem der vorstehenden Ansprüche, wobei das Erfassungssystem (2) zwei Kameras (21, 22) umfasst, wobei jede Kamera dafür eingerichtet ist, Licht aus einem der zwei Bildleiter (5, 5') zu empfangen.

16. Bildgebungsverfahren, das die Schritte umfasst des:
- Erzeugens von strukturiertem Licht,
- Leitens des erzeugten strukturierten Lichts in einem ersten Bildleiter in Aufwärtsrichtung zu einem zu beobachtenden Objekt,
- Leitens des Lichts, das von dem zu beobachtenden Objekt reflektiert wird, in einem zweiten Bildleiter in Abwärtsrichtung bis zu einem System zum Erfassen (2) des reflektierten Lichts,
- Erfassens des reflektierten Lichts mittels des Erfassungssystems,
wobei das Verfahren durch die folgenden Schritte gekennzeichnet ist, wobei jeder der zwei Bildleiter das strukturierte Licht in Aufwärtsrichtung, und das reflektierte Licht in Abwärtsrichtung leiten kann:
- selektives Lenken des erzeugten strukturierten Lichts in Aufwärtsrichtung zum ersten Bildleiter, und zur gleichen Zeit selektives Lenken des vom Objekt reflektierten Lichts in Abwärtsrichtung vom zweiten Bildleiter bis zum Erfassungssystem (2),
- selektives Lenken des erzeugten strukturierten Lichts in Aufwärtsrichtung zum zweiten Bildleiter, und zur gleichen Zeit selektives Lenken des vom Objekt reflektierten Lichts in Abwärtsrichtung vom ersten Bildleiter bis zum Erfassungssystem (2), und
- gleichzeitiges Lenken des Lichts aus den zwei Bildleitern (5, 5') in Abwärtsrichtung bis zum Erfassungssystem (2).

## Claims

1. Imaging device (E) comprising:
- at least one generator (1) of structured light,
- a first emission image guide (5, 5') for uplinking the structured light from the generator (1) to an object to be observed,
- a second return image guide (5', 5) for downlinking the light reflected by said object to be observed to a system (2) for capturing said reflected light,
the device (E) being **characterised in that** each (5, 5') of the two image guides is able to uplink the structured light and downlink the reflected light, and **in that** the device (E) comprises an optical switch (3) that can be configured into:
- a first operating mode in which it selectively directs the structured light originating from the generator (1) towards the first image guide, by uplink, and at the same time selectively directs the light reflected by the object from the second image guide, by downlink, to the capture system (2),
- a second operating mode in which it selectively directs the structured light originating from the generator (1) towards the second image guide, by uplink, and at the same time selectively directs the light reflected by the object from the first image guide, by downlink, to the capture system (2), and
- a third operating mode in which it simultaneously directs light originating from the two image guides (5, 5'), by downlink, to the capture system (2).

2. Device (E) according to claim 1, further comprising a secondary light source (8) suitable for illuminating the object.

3. Device (E) according to claim 2, further comprising an optical fibre (7) for conveying light emitted by the secondary source (8) towards the object.

4. Device (E) according to one of the previous claims, further comprising a distal tip (6) configured to diffuse light conveyed by each of the two image guides (5, 5') on the object to be observed and to redirect light reflected by said object towards each of the two image guides (5, 5').

5. Device (E) according to one of the previous claims, further comprising two distal arms, each distal arm being configured to diffuse the light conveyed by one of the image guides and to redirect the light reflected by said object towards this image guide (5, 5'), the two arms being capable of moving relative to one other so as to form a variable angle of reflection with the object to be observed as a function of the distance of the object relative to the two arms.

6. Device (E) according to one of the previous claims, wherein each image guide (5, 5') comprises a bundle of optical fibres.

7. Device (E) according to one of the previous claims, wherein each image guide (5, 5') comprises an array of mirrors and/or lenses.

8. Device (E) according to one of the previous claims, wherein the device is an endoscope.

9. Device (E) according to one of the previous claims, wherein the optical switch (3) comprises a primary mirror (30) positioned between the generator (1) and the two image guides (5, 5'), the primary mirror being suitable for enabling the selective redirection of structured light originating from the generator (1) towards a first optical axis (X1) leading to the first image guide (5) or towards a second optical axis (X2) leading to the second image guide (5').

10. Device (E) according to the previous claim, wherein the generator (1) is configured to emit structured light along the first axis (X1) leading to the first image guide (5), and the primary mirror (30) is capable of moving between:
- an active position in which the primary mirror (30) intersects the first axis (X1) between the generator (1) and the first image guide (5) and reorients towards the second image guide (5') the structured light originating from the generator (1) along the second axis (X2), and
- a passive position in which the primary mirror (30) does not intersect the first axis (X1).

11. Device (E) according to the previous claim, further comprising another mirror (33) arranged such that it redirects the light reflected by the primary mirror (31), when it is positioned in its active position, on another axis (X5) parallel to the first axis (X1), the first guide having a proximal opening aligned with the first axis (X1), and the second guide having a proximal opening aligned with the other axis (X5).

12. Device (E) according to either claim 10 or claim 11, wherein:
- the optical switch (3) comprises a first secondary mirror (31), a second secondary mirror (32),
- the first secondary mirror (31) is capable of moving between
-- an active position in which the first secondary mirror (31) intersects the first axis (X1) between the generator (1) and the first image guide (5), and reorients light originating from the first image guide (5) towards a camera (21), and
-- a passive position in which the first secondary mirror (31) does not intersect the first axis (X1),
- the second secondary mirror (32) is capable of moving between
-- an active position in which the second secondary mirror (32) intersects the second axis (X2) between the generator (1) and the primary mirror (30), and reorients light originating from the second image guide (5') towards the capture system (2), and
-- a passive position in which the first secondary mirror (31) does not intersect the first axis (X1).

13. Device (E) according to the previous claim, wherein the optical switch (3) further comprises a control unit (36) suitable for:
- in the first operating mode, positioning the primary mirror (30) and the first secondary mirror (31) in a passive position and the second secondary mirror (32) in an active position, and
- in the second operating mode, positioning the primary mirror (30) and the first secondary mirror (31) in an active position and the second secondary mirror (32) in a passive position,
- in the third operating mode, positioning the secondary mirrors (31, 32) in an active position.

14. Device (E) according to either claim 12 or claim 13, wherein the primary mirror (30) and the secondary mirrors (31, 32) are each capable of moving in translation by means of at least one bistable electromagnetic actuator.

15. Device (E) according to one of the previous claims, wherein the capture system (2) comprises two cameras (21, 22), each camera being arranged such that it receives light originating from one of the two image guides (5, 5').

16. Imaging method comprising the steps of:
- generating structured light,
- uplinking, in a first image guide, the generated structured light towards an object to be observed,
- downlinking, in a second image guide, the light reflected by said object to be observed to a system (2) for capturing said reflected light,
- capturing the reflected light by means of the capture system,
the method being **characterised by** the following steps, in which each of the two image guides are capable of uplinking the structured light and of downlinking the reflected light:
- selectively directing the generated structured light towards the first image guide, by uplink, and at the same time selectively directing the light reflected by the object from the second image guide, by downlink, to the capture system (2),
- selectively directing the generated structured light towards the second image guide, by uplink, and at the same time selectively directing the light reflected by the object from the first image guide, by downlink, to the capture system (2), and
- simultaneously directing light originating from the two image guides (5, 5'), by downlink, to the capture system (2).
